# EUROPEAN PATENT SPECIFICATION

(11) **EP 3 223 784 B1**
(45) Date of publication and mention of the grant of the patent: **14.08.2019**
(21) Application number: 14824729.9
(22) Date of filing: 19.12.2014
(51) Int. Cl.: A61K 8/46, A61Q 11/00, A61K 8/81

(54) **TOOTH WHITENING ORAL CARE PRODUCTS**
ZAHNAUFHELLENDE MUNDPFLEGEPRODUKTE
PRODUIT D'HYGIÈNE BUCCALE POUR LE BLANCHIMENT DES DENTS

(43) Date of publication of application: 04.10.2017
(73) Proprietor: Colgate-Palmolive Company, New York, NY 10022 (US)
(72) Inventor: MALONEY, Venda, Piscataway, NJ 08854 (US); CHOPRA, Suman, Monroe, NJ 08831 (US); STROTMAN, Hallena, Piscataway, NJ 08854 (US)
(74) Representative: Wibbelmann, Jobst
(86) International application number: PCT/US2014/071337
(87) International publication number: WO 2016/099524

(56) References cited:
- EP-A1- 1 935 395
- US-A1- 2012 093 905
- US-A1- 2013 129 642
- DATABASE GNPD [Online] MINTEL; May 2013 (2013-05), "Intensive Whitening Treatment Toothpaste", XP002743196, Database accession no. 2080920
- DATABASE GNPD [Online] MINTEL; February 2001 (2001-02), "New Whitening Gel Toothpaste Formulation", XP002743197, Database accession no. 10081831
- DATABASE GNPD [Online] MINTEL; October 2013 (2013-10), "White Booster Toothpste", XP002743198, Database accession no. 2242052
- DATABASE GNPD [Online] MINTEL; April 2014 (2014-04), "Whitening Gel Toothpaste", XP002743199, Database accession no. 2384129

## Description

### BACKGROUND

Many individuals are dissatisfied with their current tooth color. Thus, there is a desire for whiter teeth which can be achieved through the use of tooth whitening products. The whitening effect can be effected by chemically altering or removing the stain and/or changing the visual perception of the color of the teeth.

It is known in the literature that the visual perception of a white substance can be altered through the deposition of an optical brightener, blue pigment or blue dye, especially one for which the hue angle (in the CIELAB scale) of the reflected or emitted light is between 200 to 320 degrees. This effect is commonly used in laundry detergent products to make white clothes appear "whiter" to the human eye. The same concept has been applied to tooth whitening as well. The natural off-white or yellow color of teeth can be made to appear whiter through the deposition of a blue substance onto teeth. Using pigments with a deposition aid, such as Gantrez® (copolymers of maleic anhydride and with methyl vinylether) in toothpaste to make teeth look whiter is disclosed in EP1935395B1.

Dyes have significantly different properties than pigments, in particular, dyes are much more soluble in water than pigments. This solubility of dyes makes them much more difficult to deposit and be retained on teeth. US Patent 6,030,222 discloses depositing dyes on teeth when blended with specific carriers. US Patent Application Publication 2012/0093905 discloses dyes coupled to certain polymers.

It would be desirable to have tooth whitening oral care products containing dyes that can produce superior temporary tooth whitening effects when incorporated into oral care products.

### BRIEF SUMMARY

The present invention provides a tooth whitening oral care composition comprising:
(i) a dye having a blue to blue-violet color with a hue angle in the CIELAB system ranging from 200 to 320 degrees,
(ii) a whiteness enhancing adhesive material which is crosslinked polyvinyl pyrrolidone,
(iii) an orally acceptable carrier vehicle comprising a non-aqueous solvent,
wherein the composition comprises water in an amount of from 5 weight % to 17 weight %.

Optionally, the amount of water is from 5 weight % to 15 weight %, or from 5 weight % to less than 10 weight %, or about 7 weight %.

Optionally, the amount of water is from 10 weight % to 17 weight %.

According to the invention, the whiteness enhancing adhesive material is crosslinked polyvinyl pyrrolidone.

Optionally, the amount of whiteness enhancing adhesive material is 0.001 to 10 weight%, or 0.01 to 5 weight%, or 0.05 to 5 weight%, or 0.1 to 5 weight%, or 0.5 to 5 weight%, or 0.5 to 3 weight%, or 1 to 2 weight %, or about 1 weight% of the composition.

Optionally, the dye has a hue angle in the CIELAB system ranging from 200 to 290 degrees.

Optionally, the dye comprises from 0.001 to 2 weight%, or from 0.005 to 0.5 weight%, or from 0.01 to 0.1 weight %, or from 0.02 to 0.07 weight% of the composition.

Optionally, the dye is a blue dye such as FD&C Blue#1, FD&C Blue #2 (indigotine), D&C Blue #4, CI Food Blue 5, Acid Blue 1, or a mixture thereof.

Optionally, the dye comprises a mixture of one or more blue dyes and one or more red dyes.

Optionally, the non-aqueous solvent is glycerol.

Optionally, the composition further comprises an additional agent selected from fluoride, arginine in free or orally acceptable salt form, an antibacterial agent, an anti-inflammatory agent, and a combination of two or more thereof.

Optionally, the composition is in the form of a dentifrice.

Optionally, the composition is a toothpaste comprising one or more of an abrasive, a surfactant, a foaming agent, a vitamin, a polymer in addition to the whiteness enhancing adhesive material, an enzyme, a humectant, a thickener, an antimicrobial agent, a preservative, a flavoring, and/or a combination of two or more thereof.

Optionally, the composition is free from peroxide whitening agents.

Also disclosed is a method for whitening teeth comprising administering a composition according to the present invention to the oral cavity of a subject in need thereof. In one embodiment of the invention, the method provides a teeth whitening effect of at least a 20 % increase in tooth surface whiteness which lasts for up to four hours.

Further areas of applicability of the present invention will become apparent from the detailed description provided hereinafter.

### DETAILED DESCRIPTION

The following description of the preferred embodiment(s) is merely exemplary in nature and is in no way intended to limit the invention, its application, or uses.

### Dye

The dye should have a hue angle, h, in the CIELAB system of from 200 to 320 degrees more particularly between 250 and 290 degrees. A detailed description of hue angle may be found on p. 57 of Color Chemistry (Synthesis, Properties, and Applications of Organic Dyes and Pigments), 3rd edition by H. Zollinger published by Wiley-VCH (2001). While the preferred single dyes are blue or violet, the same effect may be achieved through mixing dyes outside of this h range; for example, such a hue angle may also be obtained by mixing a red and blue dye to yield a blue or blue-violet shaded dye. Typically, the dye is capable of reflecting sufficient light such that the treated tooth is perceivably whiter than its initial color. Preferably, the dye is colored such that its natural color is within the violet-red to green-blue color, typically from violet to blue.

Preferred dyes are water soluble dyes. The term "water-soluble" in this particular context generally means that the dye has an aqueous solubility of at least 10 g/L at 25°C, most preferably at least 100 g/L at 25°C (where the solubility is determined in un-buffered distilled water). Triarylmethane dyes are examples of water soluble dyes useful in the present invention. In some embodiments, dyes useful herein are anionic triphenylmethane dyes, and especially diaminotriphenylmethane dyes containing from two to four sulphonate groups, such as those corresponding to general formula (I): 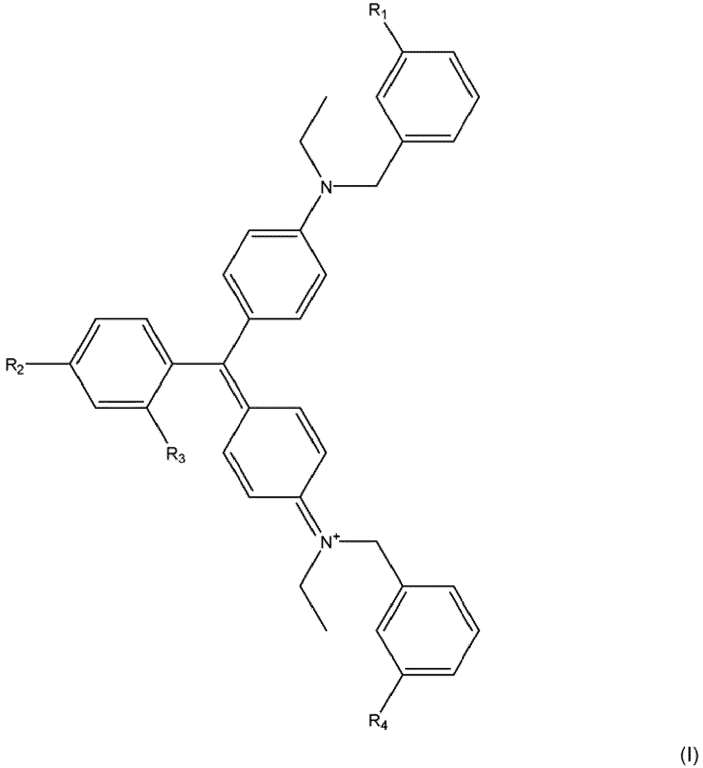 in which R₁, R₂, R₃ and R₄ are monovalent moieties which are each independently selected from hydrogen (-H), hydroxyl (-OH), halo (e.g. -Cl) and sulphonate (-SO₃⁻) groups, with the proviso that at least two of R₁ to R₁ are sulphonate groups.

An example of a dye useful herein is FD&C Blue #1, also known as Brilliant Blue FCF (Blue 1) as well as other commercial names, which corresponds to general formula (I), wherein R₂ is -H and R₁, R₃, and R₄ are sulphonate groups. FD&C Blue #1 (CAS No. [3844-45-9]) is a colorant for foods and other substances to induce a color change. It is denoted by E number E133 and has a color index of 42090 (CI 42090). It has the appearance of a reddish-blue powder. It is soluble in water, and the solution has a maximum absorption at about 628 nanometer. It is a synthetic dye produced using aromatic hydrocarbons from petroleum. It is usually a disodium salt. The diammonium salt (D&C Blue #4) has CAS No. [2650-18-2]. Calcium and potassium salts are also known. Other dyes useful herein are FD&C Blue #2 (Indigo Carmine, CI 73015, CAS No. [860-22-0]), CI Food Blue 5 (CI 42051; also known as Acid Blue 3, CAS No. [3536-49-0]), Acid Blue 1 (CI 42045, CAS No. [129-17-9]) and the like. In some embodiments, the dye is FD&C Blue#1, FD&C Blue #2, D&C Blue #4, CI Food Blue 5, Acid Blue 1, or a mixture thereof.

Mixtures of dyes also can be used even if an individual dye has a hue angle outside the desired range as long as the mixture of dyes will be within the range. For example, red dyes, e.g., FD&C Red #3, FD&C Red #40, and the like, can be used. In one embodiment FD&C Blue #1 is used in combination with FD&C Red#40.

Delta b* is a magnitude of color change along a yellow-blue axis, negative delta b* corresponding to reduced yellowness.

The amount of dye in the oral care composition can be from 0.001 to 2 weight%, from 0.002 to 1.5 weight %, from 0.003 to 1.25 weight %, from 0.004 to 1 weight %, from 0.005 to 0.5 weight%, from 0.0075 to 0.25 weight %, from 0.01 to 0.1 weight %, from 0.02 to 0.07 weight%, or from 0.03 to 0.05 weight %. In some embodiments, the amount of dye in the oral care composition is about 0.03 weight %. In some embodiments, the amount of dye in the oral care composition is about 0.05 weight %. The dye may be uniformly spread throughout the composition or, it may be dispersed in a second phase such as a stripe or other coextruded second phase. Such "dual phase" compositions have the advantage that the phases may be differently colored, presenting a more visually attractive product to the consumer.

In some embodiments, the composition is free from peroxide whitening agents. By "free from peroxide whitening agents", it is meant that the composition contains less than 0.1 weight %, less than 0.05 weight %, or less than 0.01 weight % peroxide whitening agents; or substantially no peroxide whitening agents; or that the composition contains no peroxide whitening agents. The tooth whitening effect of the oral care compositions of the present invention is provided by the presence of the dye, rather than by the presence of any peroxide whitening agents.

### Whiteness Enhancing Adhesive Materials

It has been surprisingly discovered that a number of ingredients increase dye deposition, adherence, and concomitant tooth whiteness when present in compositions of the present invention. Such ingredients are referred to herein as "whiteness enhancing adhesive materials".

### Whiteness enhancing materials in compositions containing less than 5 weight % water

In compositions which contain water in an amount of less than 5 weight %, these whiteness enhancing adhesive materials are phosphorylated acrylic polymers, or copolymers of a vinyl ether and maleic acid or maleic anhydride. In some embodiments, the copolymer of a vinyl ether and maleic acid or maleic anhydride is a copolymer of vinylmethylether and maleic acid. Unlike prior art compositions that use a dye and an adhesive material, in the compositions of the invention the dye is not covalently bonded to the whiteness enhancing adhesive material (see, e.g., US Patent Application Publication 2012/0093905).

The whiteness enhancing materials of the invention may aid the deposition of the dye onto the teeth such that tooth surface whiteness is enhanced by at least 20% or by at least 100% or by at least 400%, in comparison to the value obtained for teeth treated in an equivalent manner with a control formulation using the same amount of dye in the absence of the whiteness enhancing material. A method for determining tooth whiteness is described in the Examples.

### Whiteness enhancing materials in compositions containing 5 to 17 weight % water

It also has been surprisingly discovered that certain ingredients do not increase dye deposition, adherence, and concomitant tooth whiteness when present in compositions which comprise water in an amount of less than 5 weight %, but that these ingredients do, however, increase dye deposition and adherence (and concomitant tooth whiteness) when present in compositions which comprise water in an amount of from 5 weight % to 17 weight %. In compositions of the present invention, the whiteness enhancing adhesive material is crosslinked polyvinyl pyrrolidone (crosslinked PVP).

In some embodiments the amount of this whiteness enhancing adhesive material in the compositions of the invention is 0.001 to 10 weight%, or 0.01 to 5 weight%, or 0.05 to 5 weight%, or 0.1 to 5 weight%, or 0.5 to 5 weight%, or 0.5 to 3 weight%, or 1 to 2 weight %, or about 1 weight%.

### Stabilized dyes

The dye optionally can be stabilized. In this context "stabilized" means that the dye associated with another material, e.g., covalently bonded, bonded via weak chemical bonds, e.g., ionic bonds, hydrogen bonds, van de Waals forces and the like, or physically entrapped or entrained in or with the other material.

One means of stabilizing the dye is by covalently coupling the dyes to polymers as disclosed in US patent application 2012/0093905. Another means of stabilization is to form complexes with certain clays. Another means is to stabilize the dyes in a silica sol-gel material.

Such silica sol-gel/dye materials comprise a dye entrapped or encapsulated into a silica sol-gel. Such materials can be made by techniques known in the art, for example as disclosed in WO 2005/028604, WO 2004/081222, US 6,074,629, US 6,495,352, and US 2013/0091637.

### Silica sol-gel/dye material

Silica sol-gels refer to silicon dioxide based materials made through a sol-gel process.

Sols are formed first, which consist of a colloidal solution of very small (nanometer sized) polysiloxane particles formed through hydrolysis of the silane starting materials. Further polymerization/chemical reaction/hydrolysis converts the sols into gels by chemically linking together the individual colloidal sol particles into monolithic gels.

The sol-gel process involves low-temperature hydrolysis of suitable monomeric precursors and is suitable for encapsulation of dyes. The sol is usually formed by hydrolysis of an alkoxy silane precursor followed by condensation to yield a polymeric oxo-bridged SiO₂ network. In the process, molecules of the corresponding alcohol are liberated.

A sol can also be formed by the neutralization of an alkali metal salt of a silicate or organosiliconate with an acid.

In one embodiment a silica sol-gel matrix/dye is prepared by forming a silica sol from a solution of a silicon oxide and alkali metal oxide, such as potassium oxide or sodium oxide, in water, adjusting the pH to a pH value less than approximately 7 to stabilize the silica sol, forming a silica sol matrix solution, adding a solution containing a dye to be entrapped or encapsulated to form a silica sol matrix with entrapped dye, aging said silica sol matrix with entrapped dye, and forming a gel material.

In another embodiment a water-soluble dye to be encapsulated is dissolved in a prepared SiOx sol without using any further additives such as immobilizing or complexing agents. The dyed sol is subjected to a spray-drying process for gelling. Any solvent residues possibly present are removed by secondary drying. Starting materials suitable for the production of the spraydried sol-gels are alkoxysilane sols which are produced from unsubstituted organosilicon compounds by hydrolysis, preferably heterogeneous catalysis. Tetraethoxysilane is a preferred starting material for the production of the alkoxysilane sol. Hydrolysis of the aqueous-organic alkali silicate solutions is effected at weakly acidic pH values, preferably at pH values of 5.0 to 6.5, particularly at pH values of 5.5 to 6.0. Acidification can be effected by careful addition of acids. Hydrolysis can be effected in the form of a heterogeneous catalysis, preferably with addition of acidic ion exchangers.

### Orally Acceptable Carrier Vehicle

The oral care compositions of the invention include a vehicle or base into which the dye and whiteness enhancing adhesive material are incorporated. The carrier vehicle may be present in an amount of from 40 to 99 weight % or from 55 to 85 weight %, based on the total weight of the composition. It has been surprisingly discovered that when the dye is incorporated into a non-aqueous carrier containing little or substantially no water, the tooth whiteness effect is enhanced. In some embodiments the orally acceptable carrier vehicles comprises alcohols, polyhydric alcohols such as glycerol, sorbitol, xylitol, propylene glycol, polyols, ketones, aldehydes, carboxylic acids or salts thereof, amines, or mixtures thereof. In one embodiment the carrier comprises glycerol. Such materials typically also function as humectants.

In some aspects of the present invention, minor amounts of water are present, e.g., 5 to 17 weight %, 10 to 17 weight %, 5 to 15 weight %, 5 weight % to less than 10 weight %, 5 to 7 weight %, or about 7 weight %, about 10 weight %, or about 15 weight %.

In other aspects, the compositions comprise less than 5 weight % water, or less than 1 weight % water (the carrier being "anhydrous" when the amount of water is less than 1 weight %). For each of the ranges for the amount of water for this aspect of the invention, an alternative lower limit for the amount of water is 0.001 weight % (e.g. 0.001 weight % - less than 5 weight % or 0.001 weight % - less than 1 weight %) or 0.1 weight % (e.g. 0.1 weight % - less than 5 weight % or 0.1 weight % - less than 1 weight %).

It is surprising that the low water content of the compositions of the invention allows for enhanced delivery of the dye to the teeth, since the dyes are water soluble. It is not desired to be bound by any particular theory or mechanism, but it is believed that use of the compositions of the invention, with little or no water, drives the dye into the aqueous voids of the tooth enamel.

The orally acceptable carrier vehicle optionally can include various other ingredients which are typically incorporated into dentifrices. Examples of such other ingredients include carrier polymers, humectants, abrasives, thickener silicas or any combination of two or more thereof. The term "orally-acceptable" refers to a material or ingredient which can be applied to the oral cavity in a safe manner during normal use.

### Carrier polymers

Carrier polymers can comprise one or more anionic or nonionic polymers, and also may include additional polymers to adjust the viscosity of the formulation or enhance the solubility of other ingredients.

Suitable carrier polymers include polyethylene glycols, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Anionic polymers may be present in an amount of from about 0.001 to about 5%, more particularly about 0.01 to 5%, more particularly about 0.05 to 4%, more particularly about 0.05 to 3% of the composition. Examples of such agents are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as Gantrez®. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, (in addition to the basic amino acid polymers), e.g. as disclosed in U.S. Pat. No. 4,866,161 Sikes et al..

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Hydroxyalkyl methyl cellulose may also be present in the oral composition. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.05% to 5 weight%, more particularly about 0.5 to 5 weight% of the total composition are used. Orally acceptable carrier polymers for use in the invention are typically water soluble. Suitable orally acceptable carrier polymers for use in the invention will generally dissolve or disperse in water at a temperature of 25°C.

The amount of orally acceptable carrier vehicle polymer in compositions of the invention, whether enhancers, deposition aids, thickeners or the like, or of a combination thereof, suitably ranges from about 0.001 to 10 weight%, more particularly about 0.005 to 5 weight%, more particularly about 1 to 5 weight%, and more particularly about 1 to 3 weight%.

### Humectants

Within certain embodiments of the oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes about 15% to about 70 weight% in one embodiment or about 30% to about 65 weight% in another embodiment by weight of the dentifrice composition. Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

### Abrasives

The compositions of the invention may comprise a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate. The compositions may include one or more additional abrasives, for example silica abrasives such as precipitated silicas having a mean particle size of up to about 20 microns, such as Zeodent 115®, marketed by J. M. Huber. Other useful abrasives also include sodium metaphosphate, potassium metaphosphate, aluminum silicate, calcined alumina, bentonite or other siliceous materials, or combinations thereof. The silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason. In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns. Low oil absorption silica abrasives particularly useful in the practice of the invention are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention. The abrasive may be present in the oral care composition of the present invention at a concentration of about 10 to about 60% by weight, in other embodiment about 20 to about 45% by weight, and in another embodiment about 30 to about 50% by weight.

### Product Form

Examples of suitable product forms for compositions of the invention include dentifrices. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

### Active Agents

The effective concentration of the active ingredients for optional use herein will depend on the particular agent and the delivery system used. It is understood that a toothpaste for example will typically be diluted with water upon use, while a mouth rinse typically will not be. Thus, an effective concentration of active in a toothpaste will ordinarily be 5-15x higher than required for a mouth rinse. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product. Active agents can include one or more of a fluoride ion source, an anti-calculus agent, an amino acid, an antibacterial agent, and the like.

Arginine, where present, may be present at levels from about 1 to about 10 weight % for a consumer toothpaste or about 7 to about 20 weight % for a professional or prescription treatment product.

Fluoride where present may be present at levels of between about 25 ppm to about 25,000 ppm of fluoride ions. For consumer toothpastes, the fluoride level may be between about 500 to about 1600 ppm, or between about 500 to about 1000 ppm, or between about 1000 to about 1600 ppm, e.g., about 1100 ppm or 1450 ppm. The appropriate level of fluoride will depend on the particular application. A dentifrice or coating for professional application could have as much as about 5,000 or even about 25,000 ppm fluoride, e.g. a range of about 3,000 ppm to about 8,000 ppm fluoride.

Antibacterial agents may be included in the oral composition of the present invention and particularly noncationic halogenated diphenyl ethers agents which are desirable from considerations of effectiveness and safety, such as 2',4,4' trichloro-2 hydroxy-diphenyl ether (Triclosan) and 2,2'-dihydroxy-5,5' dibromophenyl ether. Other antibacterial agents, e.g. zinc salts, may be included in the compositions of the present invention. The antibacterial agent, when present in the oral composition, is present in concentrations of about 0.05 to about 2% by weight and preferably 0.1 to about 1% by weight. For example, a triclosan toothpaste may contain about 0.3 weight % triclosan.

Agents used to diminish teeth sensitivity such as potassium chloride, potassium nitrate, potassium citrate, dipotassium oxalate, or zinc phosphate may also be included in oral compositions of the present invention at concentrations of about 0.1 to about 10% by weight.

### Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 1000 ppm, e.g., about 1000 to about 1600 ppm, e.g., about 1100 ppm or 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride, e.g. a range of about 3,000 ppm to about 8,000 ppm fluoride.

Fluoride ion sources may be added to the compositions of the invention at a level of about 0.01 weight. % to about 10 weight. % in one embodiment or about 0.03 weight. % to about 5 weight. %, and in another embodiment about 0.1 weight. % to about 1 weight. % by weight of the composition in another embodiment. Weights of fluoride salts to provide the appropriate level of fluoride ion will obviously vary based on the weight of the counter ion in the salt.

### Foaming Agents

The oral care compositions of the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox® is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. The dosage of foaming agent in the oral care composition (i.e., a single dose) may be about 0.01 to about 0.9 % by weight, about 0.05 to about 0.5% by weight, and in another embodiment about 0.1 to about 0.2 % by weight.

### Anticalculus agents

The oral composition can include at least one anti-calculus composition, such as one or more of the anti-calculus compositions recited in U.S. Pat. No. 5,292,526 titled "Antibacterial Anti-plaque Anticalculus Oral Composition,". In various embodiments, the anti-calculus composition includes one or more polyphosphates. The anti-calculus composition can include at least one wholly or partially neutralized alkali metal or ammonium tripolyphosphate or hexametaphosphate salt present in the oral composition at an effective anti-calculus amount. The anti-calculus composition can also include at least one water soluble, linear, molecularly dehydrated polyphosphate salt effective in an anticalculus amount. The anti-calculus composition can also include a mixture of potassium and sodium salts at least one of which is present in an effective anti-calculus amount as a polyphosphate anti-calculus agent. The anti-calculus composition can also contain an effective anticalculus amount of linear molecularly dehydrated polyphosphate salt anti-calculus agent present in a mixture of sodium and potassium salts. Other useful anticalculus agents include polycarboxylate polymers and polyvinyl methyl ether/maleic anhydride (PVME/MA) copolymers, such as GANTREZ®

### Surfactants

The compositions useful in the invention may contain anionic and/or nonioinic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. For example, concentrations used for a mouthwash are typically on the order of one tenth that used for a toothpaste. In one embodiment, the anionic surfactant is present in a toothpaste at from about 0.3% to about 4.5% by weight, e.g., about 1.5%.

Nonionic surfactants include nonanionic polyoxyethylene surfactants such as Polyoxamer 407, Steareth 30, Polysorbate 20, and PEG-40 castor oil and amphoteric surfactants such as cocamiopropyl betaine (tegobaine) and cocamidopropyl betaine lauryl glucoside condensation products of ethylene oxide with various hydrogen containing compounds that are reactive therewith and have long hydorphobic chains (e.g., aliphatic chains of about 12 to 20 carbon atoms), which condensation products ("ethoxamers") contain hydrophilic polyoxyehtylene moieties, such as condensation products of poly (ethylene oxide) with fatty acids, fatty alcohols, fatty amides and other fatty moieties, and with propylene oxide and polypropylene oxides (e.g., Pluronic® materials).

The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al.. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. In a particular embodiment, the composition of the invention, e.g., Composition 1, et seq., comprises sodium lauryl sulfate.

The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

### Flavoring Agents

The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight and about 0.5 to about 1.5 weight%. The dosage of flavoring agent in the individual oral care composition dosage (i.e., a single dose) may be about 0.001 to 0.05 weight% and in another embodiment about 0.005 to about 0.015 weight%.

### Other optional ingredients

In addition to the above-described components, the embodiments of this invention can contain a variety of optional dentifrice ingredients some of which are described below. Optional ingredients include, for example, but are not limited to, adhesives, sudsing agents, sweetening agents, and additional coloring agents. These and other optional components are further described in U.S. Pat. No. 5,004,597, to Majeti; U.S. Pat. No. 3,959,458 to Agricola et al. and U.S. Pat. No. 3,937,807, to Haefele.

It is understood that while general attributes of each of the above categories of materials may differ, there may be some common attributes and any given material may serve multiple purposes within two or more of such categories of materials. All of the ingredients in the compositions may have functions in addition to their primary function, and may contribute to the overall properties of the composition, including its stability, efficacy, consistency, mouthfeel, taste, odor and so forth. Preferably, the carrier is selected for compatibility with other ingredients of the composition.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight based on the total weight of the composition. The amounts given are based on the active weight of the material. For convenience, components of the composition of invention are expressed in the singular; however it is to be understood that mixtures of components are encompassed by use of the singular expression, for example, "an orally acceptable carrier polymer" may include mixtures of two or more polymers described herein.

The invention also includes a method for whitening teeth comprising administering the composition of the invention to the oral cavity of a subject in need thereof. In one embodiment of the invention, the method provides a teeth whitening effect of at least a 20 % increase in tooth surface whiteness which lasts for up to four hours.

The invention thus provides a tooth whitening oral care composition comprising: (i) a dye having a blue to blue-violet color with a hue angle in the CIELAB system ranging from 200 to 320 degrees, (ii) a whiteness enhancing adhesive material which is crosslinked polyvinyl pyrrolidone, (iii) an orally acceptable carrier vehicle comprising a non-aqueous solvent, wherein the composition comprises water in an amount of from 5 weight% to 17 weight%; for example,
1.1. Composition 1, wherein the amount of water is from 5 weight % to 15 weight %, 5 weight % to less than 10 weight %, about 7 weight %, or 10 weight % to 17 weight %;
1.2. According to the invention, the whiteness enhancing adhesive material is crosslinked polyvinyl pyrrolidone;
1.3. Any of compositions 1 to 1.2 wherein the amount of whiteness enhancing adhesive material is 0.001 to 10 weight%, or 0.01 to 5 weight%, or 0.05 to 5 weight%, or 0.1 to 5 weight%, or 0.5 to 5 weight%, or 0.5 to 3 weight%, or 1 to 2 weight %, or about 1 weight% of the composition;
1.4. Any of compositions 1 to 1.3 wherein the dye has a hue angle in the CIELAB system ranging from 200 to 290 degrees;
1.5. Any of compositions 1 to 1.4 wherein the dye is blue dye such as FD&C Blue#1, FD&C Blue #2, D&C Blue #4, CI Food Blue 5, Acid Blue 1, or a mixture thereof;
1.6. Any of compositions 1 to 1.5 wherein the dye comprises a mixture of one or more blue dyes and one or more red dyes.
1.7. Any of compositions 1 to 1.6 wherein the dye comprises from 0.001 to less than 2 weight%, more particularly from 0.005 to 0.5 weight%, more particularly from 0.01 to 0.1 weight % and more particularly from 0.02 to 0.07 weight% of the composition;
1.8. Any of compositions 1 to 1.7 wherein the non-aqueous solvent comprises glycerol;
1.9. Any of compositions 1 to 1.8 wherein the non-aqueous solvent is glycerol;
1.10. Any of compositions 1 to 1.9 wherein the orally acceptable carrier vehicle comprises a polymer that is a synthetic anionic polymeric polycarboxylate;
1.11. Any of compositions 1 to 1.10 wherein the orally acceptable carrier comprises a polymer which is a 1:4 to 4:1 copolymers of maleic anhydride or acid with another polymerizable ethylenically unsaturated monomer;
1.12. Any of compositions 1 to 1.11 wherein the orally acceptable carrier vehicle comprises a polymer which is about 1-5 weight%, e.g., about 2 weight% of the composition;
1.13. Any of compositions 1 to 1.12 further comprising an effective amount of fluoride, e.g., wherein the fluoride is a salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof;
1.14. Any of compositions 1 to 1.13 comprising L-arginine in free or orally acceptable salt form;
1.15. Any of compositions 1 to 1.14 comprising buffering agents, e.g., sodium phosphate buffer (e.g., sodium phosphate monobasic and disodium phosphate);
1.16. Any of compositions 1 to 1.15 further comprising a humectant, e.g., selected from glycerin, sorbitol, propylene glycol, polyethylene glycol, xylitol, and mixtures thereof;
1.17. Any of compositions 1 to 1.16 further comprising an abrasive or particulate;
1.18. The immediately preceding composition wherein the abrasive or particulate is selected from sodium bicarbonate, calcium phosphate (e.g., dicalcium phosphate dihydrate), calcium sulfate, precipitated calcium carbonate, silica (e.g., hydrated silica), iron oxide, aluminum oxide, perlite, calcium silicate, mica, and combinations thereof;
1.19. Any of compositions 1.17 to 1.18 wherein the abrasive is present in an amount of about 15 weight% to about 70 weight% of the total composition weight;
1.20. Any of compositions 1 to 1.19 comprising one or more surfactants, e.g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g. in an amount of from about 0.3 weight% to about 4.5 weight%;
1.21. Any of compositions 1 to 1.20 further comprising a viscosity modifying amount of one or more polymers selected from polyethylene glycols, polysaccharides (e.g., cellulose derivatives, for example carboxymethyl cellulose, polysaccharide gums, for example xanthan gum or carrageenan gum), and combinations thereof;
1.22. Any of compositions 1 to 1.21 in the form of a dentifrice;
1.23. Any of compositions 1 to 1.22 further comprising flavoring, fragrance and/or additional coloring;
1.24. Any of compositions 1 to 1.23 comprising one or more antibacterial agents, for example comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents, phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing; e.g., comprising triclosan or cetylpyridinium chloride;
1.25. Any of compositions 1 to 1.24 further comprising an agent that interferes with or prevents bacterial attachment, e.g., solbrol or chitosan;
1.26. Any of compositions 1 to 1.25 further comprising a source of calcium and phosphate selected from (i) calcium-glass complexes, e.g., calcium sodium phosphosilicates, and (ii) calcium-protein complexes, e.g., casein phosphopeptideamorphous calcium phosphate;
1.27. Any of compositions 1 to 1.26 comprising a soluble calcium salt, e.g., selected from calcium sulfate, calcium chloride, calcium nitrate, calcium acetate, calcium lactate, and combinations thereof;
1.28. Any of compositions 1 to 1.27 further comprising a physiologically or orally acceptable potassium salt, e.g., potassium nitrate or potassium chloride, in an amount effective to reduce dentinal sensitivity;
1.29. Any of compositions 1 to 1.28 further comprising a breath freshener, fragrance or flavoring;
1.30. Any of compositions 1 to 1.29 effective upon application to the oral cavity, e.g., with brushing, to (i) reduce hypersensitivity of the teeth, (ii) to reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; and/or (xv) promote systemic health, including cardiovascular health, e.g., by reducing potential for systemic infection via the oral tissues;
1.31. A composition obtained or obtainable by combining the ingredients as set forth in any of compositions 1 to 1.29;
1.32. Any of compositions 1 to 1.31 further comprising effective amounts of additional agents selected from fluoride, 1-arginine in free or orally acceptable salt form, antibacterial agents, anti-inflammatory compounds, and whitening agents;
1.33. Any of compositions 1 to 1.32 wherein the composition is free from peroxide whitening agents.
1.34. Any of compositions 1 to 1.33 wherein the composition is a toothpaste optionally further comprising one or more of one or more of water, abrasives, surfactants, foaming agents, vitamins, polymers in addition to the whiteness enhancing adhesive material, enzymes, humectants, thickeners, antimicrobial agents, preservatives, flavorings, additional colorings and/or combinations thereof;
1.35. Any of compositions 1 to 1.34 wherein the composition is toothpaste.

Embodiments of the present invention are further described in the following examples. The examples are merely illustrative and do not in any way limit the scope of the invention as described and claimed.

### EXAMPLES

### Example 1

The formulations in accordance with this invention were evaluated for retention of blue dye on teeth from solution. The roots of human third molars were removed and the tooth was bisected from the crown through the root. Each half of the tooth was mounted in methacrylate resin and then secured in a brushing tray, enamel side facing out, using a thermal impression compound. Surface stains were removed from the teeth through brushing with a control silica toothpaste (10 min brushing with a 1:2 (w/w) slurry, 120 strokes/min). The teeth were rinsed with deionized water and cool air was used to remove excess water. The baseline color values were measured with the spectrophotometer (Spectroshade Micro, MHT technologies).

**Table 1**

| **Ingredient, weight %** | **A** | **B** | **C** | **D** |
|---|---|---|---|---|
| Glycerin | 0 | QS | QS | QS |
| Water | QS | 0 | 0 | 0 |
| FD&C Blue #1 | 0.05 | 0.05 | 0.05 | 0.05 |
| Gantrez | 0 | 0 | 2 | 0 |
| Crosslinked PVP | 0* | 0* | 0* | 2 |

| | | | | |
|---|---|---|---|---|
| *not claimed | | | | |

For the measurement of the blue retention for each formulation under test, 10 mL of the sample solution (formulations as shown in Table 1) was measured into the tray. The teeth were soaked for 2 min at room temperature. The solution was then rinsed with 100 mL of deionized water and cool air was used to remove excess water. The color values were measured with the spectrophotometer. Δb* is the change from yellow to blue with a negative value indicating a shift to blue. This is the metric by which the deposition of blue dye can be quantified. "Δb* initial" as reported in Tables 2 and 3 below is the difference between the b* values of the tooth after surface stains are removed to that after 2 min soaking with the product containing blue pigment. A change in the whiteness index (WIO) was also calculated (with WIO being calculated as described in Joiner et al, "A review of tooth colour and whiteness", Journal of Dentistry 36S (2008), S2-S7), and shows that increased deposition of blue dye results in an increase in the whiteness of a tooth. "ΔWIO initial" as reported in Tables 2 and 4 below is the change in whiteness of the tooth after 2 mins soaking with the product containing blue dye, as compared to the whiteness of the tooth after removal of surface stains with the control silica toothpaste (as described above).

The blue retention was also measured after soaking the above-treated teeth (i.e. soaked in the formulation under test for 2 mins) in artificial saliva. In this method, the above-treated teeth were soaked in 10 mL artificial saliva for 30 mins, then drained of saliva, and cool air was then used to remove excess water. The color values were again measured with the spectrophotometer. Δ b* is the change in the yellow-blue axis in CieLab color space. In Tables 2 to 4, below, the value of Δb* reported is the difference between the b* value of the tooth after removal of surface stains by brushing for 10 minutes with the 1:2 (w/w) control silica toothpaste, and the b* value for the same tooth following 2 min soaking with the blue dye-containing test product (and - for "after 30 min soak" - subsequently followed by 30 minutes of soaking in artificial saliva). Δ WIO is the change in whiteness over the same period of time (and under the same treatment conditions) as for the Δb* measurements.

**Table 2**

| | **Δb*** **initial** | **ΔWIO initial** |
|---|---|---|
| Dye control in AQ (Formulation A) | -0.9 | 3.9 |
| Dye control in non-AQ (Formulation B) | -2.7 | 11.7 |

**Table 3**

| **Non-AQ Solution** | **Δb* initial** | **Δb* after 30 min soak** |
|---|---|---|
| Dye Control (Formulation B) | -2.7 | 0.4 |
| Dye + Gantrez (Formulation C) | -3.2 | -0.5 |
| Dye + Crosslinked PVP (Formulation D) | -2.5 | 0.5 |

**Table 4**

| **Non-AQ Solution** | **ΔWIO initial** | **ΔWIO after 30 min soak** |
|---|---|---|
| Dye Control (Formulation B) | 11.7 | 1.2 |
| Dye + Gantrez (Formulation C) | 13.7 | 4.1 |
| Dye + Crosslinked PVP (Formulation D) | 10.2 | -0.7 |

From the data in Table 2, it can be seen that, by going from an aqueous (AQ) carrier system to a non-aqueous (non-AQ) carrier system for blue dye, the yellow color of teeth can be significantly reduced. This decrease in the yellow color of teeth through the deposition of blue dye resulted in an increase in the whiteness of human teeth (Tables 2, 3 and 4). The addition of Gantrez increased the retention time of the blue dye on the tooth and increased the duration of the tooth whitening provided by the blue dye system.

### Example 2

In order to investigate whether the retention of blue dye on the teeth when using crosslinked PVP (xPVP) as the whiteness-enhancing adhesive material could be increased as compared to the results obtained in Example 1, above, experiments were conducted in which the concentrations of crosslinked PVP and of water in the formulas were varied. Experiments were carried out using formulas containing 7 weight % water and either 0 weight %, 1 weight % or 2 weight % crosslinked PVP; and using formulas containing 2 weight % crosslinked PVP and either 0 weight %, 7 weight % or 30 weight % water.

The roots of human third molars were removed and the tooth was bisected from the crown through the root. Each half of the tooth was mounted in methacrylate resin and then secured in a brushing tray, enamel side facing out, using a thermal impression compound. Four teeth were mounted per tray. Surface stains were removed from the teeth through brushing with a control silica toothpaste (10 min brushing with a 1:2 (w/w) slurry). The teeth were rinsed with deionized water and cool air was used to remove excess water. The baseline color values were measured with the spectrophotometer (Spectroshade Micro, MHT technologies). The teeth were then submerged in saliva (9mL per tray) and aged at 37°C with gentle agitation for 15 mins.

For each toothpaste under test, 6g of the toothpaste was added to the tray (already containing 9mL saliva) and the teeth were brushed for 2 minutes, then rinsed with 100mL deionized water, dried with cool air, and the CIELAB color measurements were recorded. "Δb* initial" as reported in Tables 5 to 8 below is the difference between the b* value of the tooth after surface stains are removed and that after 2 min brushing with the toothpaste under test. "ΔWIO initial" as reported in Tables 5 to 8 below is the change in whiteness of the tooth after 2 mins brushing with the toothpaste under test, as compared to the whiteness of the tooth after removal of surface stains with the control silica toothpaste.

In Table 5, below, "Δb* 10 min saliva" and "Δb* 30 min saliva" were also calculated. These values correspond to the blue retention after soaking the above-treated teeth (i.e. brushed with the test toothpaste for 2 mins) in saliva for 10 minutes and 30 minutes, respectively. In this method, the above-treated teeth were soaked in 10 mL saliva for either 10 mins or 30 mins, then drained of saliva, and cool air was then used to remove excess water. The color values were again measured with the spectrophotometer. Δb* is the change in the yellow-blue axis in CieLab color space. In Table 5, below, the value of Δb* reported is the difference between the b* value of the tooth after removal of surface stains by brushing for 10 minutes with the 1:2 (w/w) control silica toothpaste, and the b* value for the same tooth following 2 min brushing with 6g of the blue dye-containing test toothpaste (and - for "10 min saliva" and "30 min saliva" - subsequently followed by 10 minutes or 30 minutes of soaking in artificial saliva). ΔWIO is the change in whiteness over the same period of time (and under the same treatment conditions) as for the Δb* measurements.

**Table 5**

| | **Δb*** | | | **ΔWIO** | | |
|---|---|---|---|---|---|---|
| | **Initial** | **10 min saliva** | **30 min saliva** | **initial** | **10 min saliva** | **30 min saliva** |
| 0 weight % xPVP* | -1.3 | 0.0 | 0.4 | 7.04 | 3.33 | 1.75 |
| 2 weight % xPVP | -1.8 | -0.8 | 0.0 | 8.40 | 5.58 | 2.57 |

| | | | | | | |
|---|---|---|---|---|---|---|
| *not claimed | | | | | | |

The compositions in Table 5, above, contained 7 weight % water and 0.05 weight % FD&C blue #1 dye. The data in this Table demonstrates that the addition of crosslinked PVP to the formula results in an increase in the retention time of the dye on the teeth.

**Table 6**

| | **Δb* initial** | **ΔWIO initial** |
|---|---|---|
| 1 weight % xPVP | -0.7 | 5.2 |
| 2 weight % xPVP | -0.6 | 2.4 |

The compositions in Table 6, above, contained 7 weight % water and 0.05 weight % FD&C blue #1 dye. The data in this Table demonstrates that 1 weight % crosslinked PVP provides optimal delivery of blue dye to the teeth.

**Table 7**

| | **Δb* initial** | **ΔWIO initial** |
|---|---|---|
| 0 weight % water | -0.56 | 3.27 |
| 7 weight % water | -1.30 | 7.04 |
| 30 weight % water | -1.30 | 6.13 |

The compositions in Table 7, above, contained 2 weight % crosslinked PVP and 0.05 weight % FD&C blue #1 dye. The data in this table demonstrates that a certain amount of water is needed in compositions containing crosslinked PVP as the whiteness enhancing adhesive material in order to activate the crosslinked PVP and enhance the delivery of the blue dye to the teeth (and therefore enhance the whiteness of the teeth).

**Table 8**

| | **Δb* initial** | **ΔWIO initial** |
|---|---|---|
| 2 weight % xPVP | -1.8 | 8.4 |
| 2 weight % Gantrez | -0.4 | 2.4 |

The compositions in Table 8, above, contained 7 weight % water and 0.05 weight % FD&C blue #1 dye. It can be seen from the results in this table that, at 2 weight % loading in a dentifrice containing 7 weight % water, crosslinked PVP provides superior blue dye delivery and retention to Gantrez.

The above results demonstrate that a formulation containing crosslinked PVP and a small amount of water can improve the retention of blue dye on teeth. The optimal level of crosslinked PVP was shown to be 1 weight %. For optimal blue dye delivery a small amount of water was required in order to activate the crosslinked PVP. An amount of 7 weight % water was found to be preferred.

### Example 3

An example of a composition according to the present invention is shown in Table 9, below:

**Table 9**

| **Ingredient** | **Target weight %** | **Weight % range** |
|---|---|---|
| Glycerin | 59.86 | 40 - 70 |
| Cleaning Silica | 20.00 | 10 - 25 |
| Water | 7.00 | 5-17 |
| Thickening silica | 4.00 | 0 - 6 |
| Polyethylene glycol | 3.00 | 1 - 5 |
| Flavor | 1.80 | 1.0 - 2.4 |
| Sodium lauryl sulfate | 1.50 | 0.75 - 2.25 |
| Polyvinylpyrrolidone | 1.00 | 0.5 - 5 |
| Titanium dioxide coated mica | 0.50 | 0.25 - 3 |
| Sodium saccharin | 0.50 | 0.25 - 0.75 |
| Xanthan gum | 0.30 | 0.1 - 0.5 |
| Sodium CMC | 0.25 | 0 - 1 |
| Sodium fluoride | 0.24 | 0.1 - 0.4 |
| FD&C Blue #1 | 0.05 | 0.02 - 2 |

## Claims

1. A tooth whitening oral care composition comprising:
(i) a dye having a blue to blue-violet color with a hue angle in the CI ELAB system ranging from 200 to 320 degrees,
(ii) a whiteness enhancing adhesive material which is crosslinked polyvinyl pyrrolidone,
(iii) an orally acceptable carrier vehicle comprising a non-aqueous solvent, wherein the composition comprises water in an amount of from 5 weight% to 17 weight%.

2. The composition of claim 1, wherein the amount of water is from 5 weight % to 15 weight %, or 10 weight % to 17 weight %, or from 5 weight % to less than 10 weight %, or about 7 weight %.

3. The composition of any of the foregoing claims, wherein the amount of whiteness enhancing adhesive material is 0.001 to 10 weight%, or 0.01 to 5 weight%, or 0.05 to 5 weight%, or 0.1 to 5 weight%, or 0.5 to 5 weight%, or 0.5 to 3 weight%, or 1 to 2 weight %, or about 1 weight% of the composition.

4. The composition of any of the foregoing claims wherein the dye comprises from 0.001 to 2 weight%, or from 0.005 to 0.5 weight%, or from 0.01 to 0.1 weight %, or from 0.02 to 0.07 weight% of the composition.

5. The composition of any of the foregoing claims wherein the dye is a blue dye such as FD&C Blue# 1, FD&C Blue # 2, D&C Blue # 4, CI Food Blue 5, Acid Blue 1, or a mixture thereof; and/or wherein the dye comprises a mixture of one or more blue dyes and one or more red dyes.

6. The composition of any of the foregoing claims further comprising an additional agent selected from fluoride, arginine in free or orally acceptable salt form, an antibacterial agent, an anti-inflammatory agent, and a combination of two or more thereof.

7. The composition of any of the foregoing claims wherein the composition is a toothpaste comprising one or more of an abrasive, a surfactant, a foaming agent, a vitamin, a polymer in addition to the whiteness enhancing adhesive material, an enzyme, a humectant, a thickener, an antimicrobial agent, a preservative, a flavoring, and/or a combination of two or more thereof.

8. The composition of any of the foregoing claims, wherein the composition is free from peroxide whitening agents.

## Patentansprüche

1. Zahnaufhellende Mundpflegezusammensetzung, die umfasst:
(i) einen Farbstoff mit einer blau bis blauvioletten Farbe mit einem Farbtonwinkel im CIELAB-System im Bereich von 200 bis 320 Grad,
(ii) ein weißverstärkendes Haftmaterial, das vernetztes Polyvinylpyrrolidon ist,
(iii) ein oral unbedenkliches Trägervehikel, das ein nichtwässriges Lösungsmittel umfasst, wobei die Zusammensetzung Wasser in einer Menge von 5 Gew.-% bis 17 Gew.-% umfasst.

2. Zusammensetzung nach Anspruch 1, wobei die Menge an Wasser 5 Gew.-% bis 15 Gew.-% oder 10 Gew.-% bis 17 Gew.-% oder 5 Gew.-% bis weniger als 10 Gew.-% oder ungefähr 7 Gew.-% beträgt.

3. Zusammensetzung nach einem der vorstehenden Ansprüche, wobei die Menge an weißverstärkendem Haftmaterial 0,001 bis 10 Gew.-% oder 0,01 bis 5 Gew.-% oder 0,05 bis 5 Gew.-% oder 0,1 bis 5 Gew.-% oder 0,5 bis 5 Gew.-% oder 0,5 bis 3 Gew.-% oder 1 bis 2 Gew.-% oder ungefähr 1 Gew.-% der Zusammensetzung ausmacht.

4. Zusammensetzung nach einem der vorstehenden Ansprüche, wobei der Farbstoff 0,001 bis 2 Gew.-% oder 0,005 bis 0,5 Gew.-% oder 0,01 bis 0,1 Gew.-% oder 0,02 bis 0,07 Gew.-% der Zusammensetzung umfasst.

5. Zusammensetzung nach einem der vorstehenden Ansprüche, wobei der Farbstoff ein blauer Farbstoff wie z. B. FD&C Blue # 1, FD&C Blue # 2, D&C Blue # 4, CI Food Blue 5, Acid Blue 1 oder eine Mischung dieser ist; und/oder wobei der Farbstoff eine Mischung aus einem oder mehreren blauen Farbstoffen und einem oder mehreren roten Farbstoffen umfasst.

6. Zusammensetzung nach einem der vorstehenden Ansprüche, die ferner ein zusätzliches Mittel umfasst, das aus Fluorid, Arginin in freier Form oder in Form eines oral unbedenklichen Salzes, einem antibakteriellen Mittel, einem Entzündungshemmer und einer Kombination von zwei oder mehr davon ausgewählt ist.

7. Zusammensetzung nach einem der vorstehenden Ansprüche, wobei die Zusammensetzung eine Zahnpasta ist, die eines oder mehrere von einem Schleifmittel, einem Tensid, einem Schaumbildner, einem Vitamin, einem Polymer zusätzlich zum weißverstärkenden Haftmaterial, einem Enzym, einem Feuchthaltemittel, einem Verdickungsmittel, einem antimikrobiellen Mittel, einem Konservierungsmittel, einem Geschmackstoff und/oder eine Kombination von zwei oder mehr dieser umfasst.

8. Zusammensetzung nach einem der vorstehenden Ansprüche, wobei die Zusammensetzung frei von Peroxidaufhellern ist.

## Revendications

1. Composition d'hygiène buccale pour le blanchiment des dents, comprenant :
(i) un colorant ayant une couleur de bleu à bleu-violet avec un angle de teinte dans le système CIELAB se situant dans la plage de 200 à 320 degrés ;
(ii) une matière adhésive améliorant la blancheur, qui est de la polyvinyl pyrrolidone réticulée ;
(iii) un véhicule support acceptable par voie orale comprenant un solvant non aqueux,
la composition comprenant de l'eau dans une quantité de 5 % en poids à 17 % en poids.

2. Composition selon la revendication 1, dans laquelle la quantité d'eau est de 5 % en poids à 15 % en poids, ou de 10 % en poids à 17 % en poids, ou de 5 % en poids à moins de 10 % en poids, ou d'environ 7 % en poids.

3. Composition selon l'une quelconque des revendications précédentes, dans laquelle la quantité de matière adhésive améliorant la blancheur est de 0,001 à 10 % en poids, ou de 0,01 à 5 % en poids, ou de 0,05 à 5 % en poids, ou de 0,1 à 5 % en poids, ou de 0,5 à 5 % en poids, ou de 0,5 à 3 % en poids, ou de 1 à 2 % en poids, ou d'environ 1 % en poids, de la composition.

4. Composition selon l'une quelconque des revendications précédentes, dans laquelle le colorant comprend de 0,001 à 2 % en poids, ou de 0,005 à 0,5 % en poids, ou de 0,01 à 0,1 % en poids, ou de 0,02 à 0,07 % en poids, de la composition.

5. Composition selon l'une quelconque des revendications précédentes, dans laquelle le colorant est un colorant bleu tel que FD&C Blue #1, FD&C Blue #2, D&C Blue #4, CI Food Blue 5, Acid Blue 1, ou un mélange de ceux-ci ; et/ou dans laquelle le colorant comprend un mélange d'au moins un colorant bleu et d'au moins un colorant rouge.

6. Composition selon l'une quelconque des revendications précédentes, comprenant en outre un agent supplémentaire choisi parmi un fluorure, l'arginine dans une forme libre ou de sel acceptable par voie orale, un agent antibactérien, un agent anti-inflammatoire, et une combinaison d'au moins deux de ceux-ci.

7. Composition selon l'une quelconque des revendications précédentes, dans laquelle la composition est une pâte dentifrice comprenant au moins l'un parmi un abrasif, un tensio-actif, un agent moussant, une vitamine, un polymère en plus de la matière adhésive améliorant la blancheur, une enzyme, un humectant, un épaississant, un agent antimicrobien, un conservateur, un agent aromatisant et/ou une combinaison d'au moins deux de ceux-ci.

8. Composition selon l'une quelconque des revendications précédentes, dans laquelle la composition est exempte d'agents de blanchiment de type peroxyde.
